# EUROPÄISCHE PATENTSCHRIFT

(11) **EP 1 133 266 B1**
(45) Veröffentlichungstag und Bekanntmachung des Hinweises auf die Patenterteilung: **02.06.2004**
(21) Anmeldenummer: 99944213.0
(22) Anmeldetag: 27.09.1999
(51) Int. Cl.: A61F 2/36

(54) **FEMURKOMPONENTE FÜR EINE HÜFTENDOPROTHESE**
FEMUR COMPONENT FOR A HIP ENDOPROSTHESIS
PARTIES DE FEMUR POUR ENDOPROTHESE DE HANCHE

(43) Veröffentlichungstag der Anmeldung: 19.09.2001
(73) Patentinhaber: Biomet Merck GmbH, 3216 Ried b. Kerzers (CH)
(72) Erfinder: BÄTSCHER, Cyrill, Carl, CH-4456 Tenniken (CH); KOCH, Rudolf, CH-4436 Oberdorf (CH)
(74) Vertreter: Lusuardi, Werther Giovanni, Dr.
(86) Internationale Anmeldenummer: PCT/CH1999/000456
(87) Internationale Veröffentlichungsnummer: WO 2001/022904

(56) Entgegenhaltungen:
- EP-A- 0 821 923
- WO-A-89/08436
- WO-A-95/12369
- FR-A- 2 602 672
- FR-A- 2 667 784

## Beschreibung

Die Erfindung betrifft eine Femurkomponente für eine Hüftendoprothese gemäss dem Oberbegriff des Patentanspruchs 1.

Aus der EP-A 0 821 923 ist eine derartige Femurkomponente bekannt. Die Nachteile dieses Prothesenschaftes besteht darin, dass die auf dem Schaft angebrachten Längsrillen gegen die Schaftspitze hin, d.h. gegen distal hin konvergieren. Eine Spongiosaverdichtung findet somit nur in dem von der Keilform des Schaftkörpers gebildeten Masse statt.

Aus der WO89108436 ist eine weitere Femurkomponente bekannt, welche im proximalen Teil des Schaftes eine Anzahl winkelförmiger Rippen aufweist, welche aber untereinander parallel angeordnet sind. Die in sich geschlossenen, winkelförmigen Rippen mit einem Öffnungswinkel der beiden Schenkel von etwa 90° sind für die Spongiosaverdichtung ungünstig.

Hier will die Erfindung Abhilfe schaffen. Der Erfindung liegt das Problem zugrunde, eine Femurkomponente zu schaffen, welche bei ihrer Einführung in den vorbereiteten Markraumkanal zu einer zusätzlichen Verdichtung der Spongiosa führt.

Die Erfindung löst die gestellte Aufgabe mit einer Femurkomponente, welche die Merkmale des Anspruchs 1 aufweist.

Damit ist der Vorteil erzielbar, dass eine Erhöhung der Primärstabilität des unzementiert implantierten Schaftes erreicht wird. Ferner hat sich gezeigt, dass verdichtete Spongiosa im Anwachsverhalten an die poröse Oberflächenstruktur von künstlichen Hüftprothesenschäften stimuliert wird, was sich in einer rascheren und innigeren Sekundärstabilität des Schaftes äussert.

Die durch die Erfindung erreichten Vorteile sind im wesentlichen darin zu sehen, dass dank der erfindungsgemässen Femurkomponente eine verbesserte Selbstzentrierung des Schaftes im durch das Vorraspeln ausgeweiteten Knochenbett stattfindet, was Fehlstellungen des Schaftes, wie Varus- oder Valgus-Positionen minimiert.

Bei einer bevorzugten Weiterbildung der Erfindung beträgt der Divergenzwinkel der Längsrippen unter sich 1° bis 2°. Die Längsrippen können auch einen proximal gelegenen gemeinsamen Schnittpunkt aufweisen. Ist dies der Fall, nimmt der jeweilige Längsrippen-Divergenzwinkel bezogen auf die Schaftachse nach medial und lateral zu, was zur Folge hat, dass auch bei kürzer werdenden Rippen eine ausreichende Spongiosaverdichtung stattfindet.

Der Schaftteil weist medial von der Zentralachse aus gesehen, mindestens drei Längsrippen auf und lateral von der Zentralachse aus gesehen mindestens eine Längsrippe auf.

Vorzugsweise verjüngt sich der Schaftteil gegen sein Ende hin, z.B. konisch, keilförmig, sphärisch oder prismatisch. Bei einer keilförmigen Verjüngung schliesst die anteriore Seite und die posteriore Seite des Schaftteiles einen Winkel von vorzugsweise 0,5° bis 3,0° ein.

Die Längsrippen weisen vorzugsweise eine Höhe im Bereich von 1 bis 3 mm auf. Der minimalen Abstand der Längsrippen untereinander beträgt zweckmässigerweise 1 mm und der maximale Abstand 5 mm. Die minimale Länge der Längsrippen beträgt zweckmässigerweise 10 mm und die maximale Länge 130 mm. Vorzugsweise sind die Längsrippen als Lamellen mit konstanter Dicke im Bereich von 0,4 bis 1,0 mm ausgebildet.

Die Erfindung und Weiterbildungen der Erfindung werden im folgenden anhand der teilweise schematischen Darstellung eines Ausführungsbeispiels noch näher erläutert.

Es zeigt:

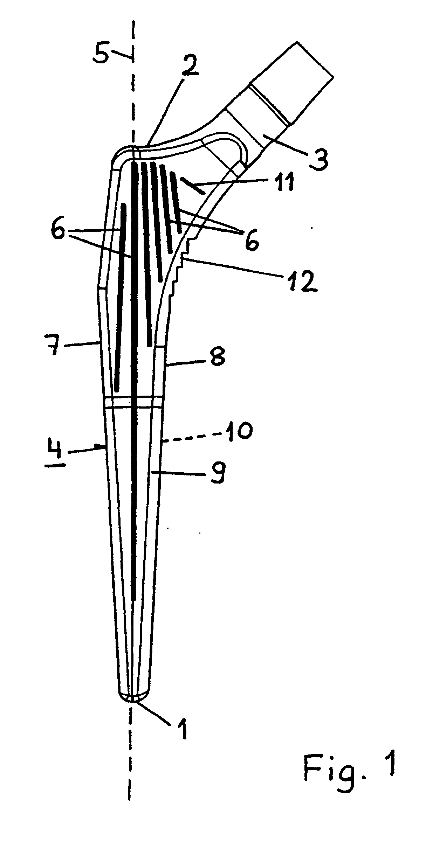

Fig. 1 eine Seitenansicht der Femurkomponente.

Die in Fig. 1 dargestellte Femurkomponente für eine Hüftendoprothese besitzt ein distales, zur Einführung in den Markraum des Femur bestimmtes Ende 1 und eine proximale Schulterpartie 2 mit nach medial gerichtetem Hals 3, an welchem ein (nicht gezeichneter) Kugelkopf befestigbar ist.

Zwischen der Schulterpartie 2 und dem distalen Ende 1 erstreckt sich ein Schaftteil 4 mit Zentralachse 5, lateraler Seite 7, medialer Seite 8, anteriorer Seite 9 und posteriorer Seite 10.

Auf der anterioren Seite 9 und posterioren Seite 10 des Schaftteiles 4 sind sechs Längsrippen 6 in Form von Lamellen konstanter Dicke (z.B. 0,7 mm) angebracht. Eine der Längsrippen stimmt mit der Richtung der Zentralachse 5 überein, eine Längsrippe ist lateral davon angeordnet und vier Längsrippen befinden sind medial von der Zentralachse. Die Längsrippen weisen, bezogen auf die Zentralachse 5, von proximal nach distal eine Divergenz auf. Der Divergenzwinkel zwischen zwei benachbarten Längsrippen 6 beträgt 2°, d.h. die zentrale Rippe weist einen Winkel von 0° zur Zentralachse 5 auf, die 1. Längsrippe medial sowie lateral weisen einen Winkel von 2° zur Zentralachse 5 auf, die 2. Rippe medial einen Winkel von 4° und die 3. Rippe medial von 6°.

Der Schaftteil 4 verjüngt sich gegen das Ende 1 hin keilförmig.

Die medial von der medialsten Längsrippe 6 auf dem Schaftteil 4 angeordnete Marke 11 dient lediglich der Markierung der Resektionsebene und stellt keine Längsrippe dar.

Die am medialen Bogen des Schaftteils 4 angeordneten Zacken 12 sollen ein Absinken der Femurkomponenten nach erfolgter Implantation verhindern.

## Patentansprüche

1. Femurkomponente für eine Hüftendoprothese mit
A) einem distalen, zur Einführung in den Markraum des Femur bestimmten Ende (1),
B) einer proximalen Schulterpartie (2) mit nach medial gerichtetem Hals (3), an welchem ein Kugelkopf befestigbar ist,
C) einem sich zwischen Schulterpartie (2) und dem distalen Ende (1) erstreckenden Schaftteil (4) mit Zentralachse (5), lateraler Seite (7), medialer Seite (8), anteriorer Seite (9) und posteriorer Seite (10) sowie
D) einer Anzahl Längsrippen (6), welche auf der anterioren Seite (9) und posterioren Seite (10) des Schaftteiles (4) angebracht sind,
**dadurch gekennzeichnet, dass**
E) die einzelnen Längsrippen (6) relativ zueinander und bezogen auf die Zentralachse (5) von proximal nach distal eine Divergenz aufweisen; und
F) der Divergenzwinkel der Längsrippen (6) unter sich 0,5° bis 5,0° beträgt.

2. Femurkomponente nach Anspruch 1, **dadurch gekennzeichnet, dass** der Divergenzwinkel der Längsrippen (6) unter sich 1° bis 2° beträgt.

3. Femurkomponente nach Anspruch 1 oder 2, **dadurch gekennzeichnet, dass** eine der Längsrippen (6) mit der Richtung der Zentralachse (5) übereinstimmt.

4. Femurkomponente nach einem der Ansprüche 1 bis 3, **dadurch gekennzeichnet, dass** die Längsrippen (6) einen proximal gelegenen gemeinsamen Schnittpunkt aufweisen.

5. Femurkomponente nach einem der Ansprüche 1 bis 4, **dadurch gekennzeichnet, dass** der Schaftteils (4) medial von der Zentralachse (5) aus gesehen mindestens 3 Längsrippen (6) aufweist.

6. Femurkomponente nach einem der Ansprüche 1 bis 5, **dadurch gekennzeichnet, dass** der Schaftteils (4) lateral von der Zentralachse (5) aus gesehen mindestens eine Längsrippe (6) aufweist.

7. Femurkomponente nach einem der Ansprüche 1 bis 6, **dadurch gekennzeichnet, dass** der Schaftteil (4) sich gegen das Ende (1) hin verjüngt.

8. Femurkomponente nach Anspruch 7, **dadurch gekennzeichnet, dass** der Schaftteil (4) sich gegen das Ende (1) hin konisch verjüngt.

9. Femurkomponente nach Anspruch 7, **dadurch gekennzeichnet, dass** der Schaftteil (4) keilförmig ausgebildet ist.

10. Femurkomponente nach Anspruch 9, **dadurch gekennzeichnet, dass** die anteriore Seite (9) und die posteriore Seite (10) des Schaftteiles (4) einen Winkel von 0,5° bis 3,0° einschliessen.

11. Femurkomponente nach einem der Ansprüche 1 bis 10, **dadurch gekennzeichnet, dass** die Längsrippen (6) eine Höhe im Bereich von 1 - 3 mm aufweisen.

12. Femurkomponente nach einem der Ansprüche 1 bis 11, **dadurch gekennzeichnet, dass** die Längsrippen (6) untereinander einen minimalen Abstand von 1 mm aufweisen.

13. Femurkomponente nach einem der Ansprüche 1 bis 12, **dadurch gekennzeichnet, dass** die Längsrippen (6) untereinander einen maximalen Abstand von 5 mm aufweisen.

14. Femurkomponente nach einem der Ansprüche 1 bis 13, **dadurch gekennzeichnet, dass** die Längsrippen (6) eine minimale Länge von 10 mm aufweisen.

15. Femurkomponente nach einem der Ansprüche 1 bis 14, **dadurch gekennzeichnet, dass** die Längsrippen (6) eine maximale Länge von 130 mm aufweisen.

16. Femurkomponente nach einem der Ansprüche 1 bis 15, **dadurch gekennzeichnet, dass** die Längsrippen (6) als Lamellen mit konstanter Dicke ausgebildet sind.

17. Femurkomponente nach Anspruch 16, **dadurch gekennzeichnet, dass** die Dicke der Lamellen im Bereich von 0,4 bis 1,0 mm liegt.

## Claims

1. Femur component for a hip endoprosthesis with
A) a distal end (1) intended for insertion into the marrow cavity of the femur,
B) a proximal shoulder section (2) with a neck (3) running in the medial direction to which a spherical head is attached,
C) a shaft section (4) reaching between the shoulder section (2) and the distal end (1) with central axis (5), lateral side (7), medial side (8), anterior side (9) and posterior side (10),
D) a number of longitudinal ribs (6) that are attached to the anterior side (9) and posterior side (10) of the shaft section (4)
**characterised in that**
E) the individual longitudinal ribs (6) show a divergence from proximal to distal in relation to each other and in relation to the central axis (5), and
F) the divergence angle of the longitudinal ribs (6) to each other measures 0.5° to 5.0°.

2. Femur component according to Claim 1, **characterised in that** the divergence angle of the longitudinal ribs (6) to each other measures 1° to 2°,

3. Femur component according to Claim 1 or Claim 2, **characterised in that** one of the longitudinal ribs (6) conforms to the direction of the central axis (5),

4. Femur component according to one of the claims 1 to 3, **characterised in that** the longitudinal ribs (6) have a proximal common intersection,

5. Femur component according to one of the claims 1 to 4, **characterised in that** the shaft section (4) has at least three longitudinal ribs (6) medial as seen from the central axis (5),

6. Femur component according to one of the claims 1 to 5, **characterised in that** the shaft section (4) has at least one longitudinal rib (6) lateral as seen from the central axis (5),

7. Femur component according to one of the claims 1 to 6, **characterised in that** the shaft section (4) tapers towards the end (1),

8. Femur component according to Claim 7, **characterised in that** the shaft section (4) tapers conical towards the end (1),

9. Femur component according to Claim 7, **characterised in that** the shaft section (4) is formed as a wedge,

10. Femur component according to Claim 9, **characterised in that** the anterior side (9) and the posterior side (10) of the shaft section (4) form an angle of 0.5° to 3.0°,

11. Femur component according to one of the claims 1 to 10, **characterised in that** the longitudinal ribs (6) have a height in the range 1 - 3 mm,

12. Femur component according to one of the claims 1 to 11, **characterised in that** the longitudinal ribs (6) have a minimum distance to each other of 1 mm,

13. Femur component according to one of the claims 1 to 12, **characterised in that** the longitudinal ribs (6) have a maximum distance to each other of 5 mm,

14. Femur component according to one of the claims 1 to 13, **characterised in that** the longitudinal ribs (6) have a minimum length of 10 mm,

15. Femur component according to one of the claims 1 to 14, **characterised in that** the longitudinal ribs (6) have a maximum length of 130 1 mm,

16. Femur component according to one of the claims 1 to 15, **characterised in that** the longitudinal ribs (6) are formed as lamella or uniform thickness,

17. Femur component according to Claim 16, **characterised in that** the thickness of the lamellas is in the range 0.4 to 1.0 mm.

## Revendications

1. Constituant fémoral pour une endoprothèse de la hanche avec
A) une extrémité distale (1) destinée à être introduite dans le canal médullaire du fémur,
B) un épaulement proximal (2) avec un col (3) orienté médialement et sur lequel on peut fixer une tête sphérique,
C) une tige (4) qui s'étend entre l'épaulement (2) et l'extrémité distale (1) et présente un axe central (5), une face latérale (7), une face médiale (8), une face antérieure (9) et une face postérieure (10) ainsi que
D) un nombre de nervures longitudinales (6) placées sur la face antérieure (9) et la face postérieure (10) de la tige (4),
**caractérisé en ce que**
E) les nervures longitudinales (6) présentent entre elles et par rapport à l'axe central (5) une divergence, depuis le côté proximal en direction du côté distal; et **en ce que**
F) l'angle de divergence des nervures longitudinales (6) entre elles est de 0,5° à 5,0°.

2. Constituant fémoral selon la revendication 1, **caractérisé en ce que** l'angle de divergence des nervures longitudinales (6) entre elles est de 1° à 2°.

3. Constituant fémoral selon la revendication 1 ou 2, **caractérisé en ce qu'**une des nervures longitudinales (6) correspond à la direction de l'axe central (5).

4. Constituant fémoral selon une des revendications 1 à 3, **caractérisé en ce que** les nervures longitudinales (6) présentent un point d'intersection commun proximal.

5. Constituant fémoral selon une des revendications 1 à 4, **caractérisé en ce que** la tige (4) présente au moins 3 nervures longitudinales (6) médialement en partant de l'axe central (5).

6. Constituant fémoral selon une des revendications 1 à 5, **caractérisé en ce que** la tige (4) présente au moins une nervure longitudinale (6) latéralement en partant de l'axe central (5).

7. Constituant fémoral selon une des revendications 1 à 6, **caractérisé en ce que** la tige (4) se rétrécit vers l'extrémité (1).

8. Constituant fémoral selon la revendication 7, **caractérisé en ce que** la tige (4) se rétrécit vers l'extrémité (1) et devient conique.

9. Constituant fémoral selon la revendication 7, **caractérisé en ce que** la tige (4) est cunéiforme.

10. Constituant fémoral selon la revendication 9, **caractérisé en ce que** la face antérieure (9) et la face postérieure (10) de la tige (4) forment un angle de 0,5° à 3,0°.

11. Constituant fémoral selon une des revendications 1 à 10, **caractérisé en ce que** les nervures longitudinales (6) présentent une hauteur de 1 à 3 mm.

12. Constituant fémoral selon une des revendications 1 à 11, **caractérisé en ce que** les nervures longitudinales (6) présentent entre elles un écart minimal de 1 mm.

13. Constituant fémoral selon une des revendications 1 à 12, **caractérisé en ce que** les nervures longitudinales (6) présentent entre elles un écart maximal de 5 mm.

14. Constituant fémoral selon une des revendications 1 à 13, **caractérisé en ce que** les nervures longitudinales (6) présentent une longueur minimale de 10 mm.

15. Constituant fémoral selon une des revendications 1 à 14, **caractérisé en ce que** les nervures longitudinales (6) présentent une longueur maximale de 130 mm.

16. Constituant fémoral selon une des revendications 1 à 15, **caractérisé en ce que** les nervures longitudinales (6) ont la forme de lamelles d'épaisseur constante.

17. Constituant fémoral selon la revendication 16, **caractérisé en ce que** l'épaisseur des lamelles se situe dans une plage de 0,4 à 1,0 mm.
